# EUROPEAN PATENT APPLICATION

(11) **EP 4 350 349 A1**
(43) Date of publication of application: **10.04.2024**
(21) Application number: 22816094.1
(22) Date of filing: 31.05.2022
(51) Int. Cl.: G01N 33/49, G01N 33/68

(54) **METHOD FOR EVALUATING RISK OF ACUTE CEREBRAL VASCULAR DISEASE USING SOLUBLE CLEC2**

(30) Priority: 31.05.2021 JP 2021091606
(71) Applicant: PHC Corporation, Toon-shi Ehime 791-0395 (JP)
(72) Inventor: WADA, Hideo, Yokkaichi-shi, Mie 510-8561 (JP); KAWAMURA, Masahide, Toon-shi, Ehime 791-0395 (JP)
(74) Representative: Gille Hrabal Partnerschaftsgesellschaft mbB Patentanwälte
(86) International application number: PCT/JP2022/022076
(87) International publication number: WO 2022/255348

(57) **Abstract**

Provided is a convenient, rapid, and objective blood test-based risk evaluation method for the diagnosis of TIA that can complement the clinical symptom interview, and provided is a convenient, rapid, and objective blood test-based risk evaluation method for the differential diagnosis of cardiogenic and non-cardiogenic acute cerebral vascular diseases such as cerebral infarction and TIA that can complement imaging, electrocardiogram, and conventional blood tests. The concentration of soluble CLEC2 is measured in the blood collected from a patient.

## Description

### TECHNICAL FIELD

The present invention relates to a method for evaluating the risk of acute cerebral vascular disease using soluble CLEC2.

### BACKGROUND ART

Stroke is a group of diseases that includes ischemic stroke which is caused by a clogged blood vessel in the brain, i.e., cerebral infarction, hemorrhagic stroke which is caused by a rupture of blood vessel in the brain, i.e., cerebral hemorrhage, and subarachnoid hemorrhage, and is the second leading cause of death worldwide, after ischemic heart disease. Stroke is not only a major cause of death, but even after patients survive, they are often left with severe aftereffects and require nursing care, which places a heavy burden on patients and society. Therefore, a system that enables prevention, early diagnosis, prompt response after diagnosis, and care and risk management by a wide range of medical personnel is needed.

Among ischemic strokes, cerebral infarction occurs when blood vessels in the brain become narrowed or a blood clot formed elsewhere clogs blood vessels in the brain, preventing the brain from receiving oxygen and nutrients, resulting in necrosis of brain nerve cells and various disorders.

Thrombi that cause cerebral infarction are roughly classified into two types: cardiogenic cerebral embolism, in which a thrombus formed in the heart travels through blood vessels and clogs arteries in the brain, and noncardiogenic cerebral infarction, in which a thrombus formed outside the heart causes a cerebral infarction. In the case of cardiogenic embolism, fibrin is considered to be the main component of thrombus, and anticoagulants (warfarin and DOACs) that inhibit fibrin formation are used for treatment and secondary prevention. On the other hand, in the case of non-cardiogenic cerebral infarction, it is believed that the thrombus is mainly composed of platelets, and antiplatelet agents (aspirin, clopidogrel, or the like) that inhibit platelet activation are used for treatment and secondary prevention. In other words, it is important to correctly diagnose whether the stroke is cardiogenic or non-cardiogenic, because the treatment for cardiogenic ischemic stroke is clearly different from that for non-cardiogenic stroke.

Cardiogenic cerebral infarction forms a relatively large infarct caused by a thrombus formed by stasis or the like of blood flow in the atria due to atrial fibrillation, which travels to the brain and causes an infarction. The diagnosis is based on the observation of large infarcts on MRI images, and information such as atrial fibrillation, arrhythmia, pre-existing cardiac diseases, and the like. On the other hand, non-cardiogenic cerebral infarction is classified into atheromatous, lacunar types, and the like. Since atherosclerotic cerebral infarction clogs relatively large arteries and lacunar stroke clogs small vessels, the diagnosis is made based on information such as the size of the infarct on MRI images, the state of arteriosclerosis, metabolic abnormalities or the like on blood tests.

Atrial fibrillation is detected by electrocardiogram, but it can be persistent or paroxysmal, and paroxysmal cases are difficult to detect easily. In many cases, it is not possible to clearly distinguish between atherosclerotic and cardiogenic cerebral infarction.

On the other hand, transient ischemic attack (TIA) is known as a cerebral infarction-like symptom. TIA is a condition in which a blood vessel is narrowed or occluded by a thrombus as in cerebral infarction, but the event is transient and symptoms similar to those of cerebral infarction occur transiently.

TIA was once considered to be the disappearance of ischemic symptoms in the brain within 24 hours. Since nowadays, advances in imaging technology have led to an increase in the number of cases of cerebral infarction detected by imaging even after symptoms have disappeared, a 2009 American Heart Association, American Stroke Association (AHA/ASA) paper defines TIA as "a transient episode of neurological dysfunction caused by focal brain, spinal cord, or retinal ischemia, without acute infarction," and distinguishes infarction and TIA as separate entities, regardless of the duration of symptoms. In other words, cerebral infarction is defined as the presence of infarction on imaging even if symptoms have disappeared, while TIA is defined as the absence of symptoms and the absence of cerebral infarction on imaging. The International Classification of Diseases (ICD-11, 2018) of the World Health Organization (WHO) also states that TIA must not have an infarcted foci, and there is a global consensus that TIA should be judged by imaging.

In Japan, the Japanese Stroke Association defined in 2019 as "a transient episode of neurological dysfunction caused by focal cerebral or retinal ischemia, with no evidence of acute infarction. The episodes of neurological dysfunction should disappear within 24 hours at the most." It is also clarified that although TIA is a type of cerebrovascular diseases, it is defined as a stroke that does not present with a cerebral infarcted foci, and thus is not included in the stroke category, which consists of three diseases, i.e., cerebral infarction, cerebral hemorrhage, and subarachnoid hemorrhage.

In this way, TIA is clearly distinguished from cerebral infarction as a separate disease. The diagnosis of TIA is extremely important even when the symptoms have disappeared. This is because TIA patients often develop cerebral infarction at an early stage. Clinical studies have shown that 10% to 15% of patients with TIA developed cerebral infarction within 3 months, and half of them within 48 hours, indicating that cerebral infarction often occurs much earlier than previously thought. Based on such data, the Japan Stroke Society recommends in its "Guidelines for Stroke Treatment" issued in 2009 that "prophylactic treatment should be started immediately if TIA is suspected." In other words, TIA is said to be a precursor of cerebral infarction, which is a serious disease.

As mentioned above, the presence or absence of a lesion is necessary for the diagnosis of TIA, and the longer the time from the onset of TIA until the imaging test by the diffusion weighted image (DWI) of MRI is performed, the higher the DWI positive rate (17% within 6 hours and 37% after 24 hours). In addition, it is said that many patients with no lesions on the first DWI will have lesions on the second DWI. In other words, in the case that cerebral infarction is defined when a lesion is detected on imaging, and TIA is defined when no lesion is detected, the timing of DWI has a significant impact on the diagnosis.

However, there is a need for a method that enables not only specialized hospitals but also general practitioners who cannot perform imaging tests to diagnose TIA, and it is also necessary to respond to situations in which physicians who are not specialists such as stroke specialists or neurologists have to make quick decisions in the emergency rooms of general hospitals.

In addition, it is often difficult to make a clear diagnosis of TIA because the symptoms of TIA often disappear at the time of the patient's first visit to the hospital, and the diagnosis of TIA in this case depends on an accurate history of the patient's condition, including medical history and complications. In addition, the diseases that should be differentiated from TIA include migraine with neurological symptoms, seizures, idiopathic transient global amnesia, Meniere's syndrome, hyperventilation syndrome, syncope, hypoglycemia, narcolepsy, periodic tetraplegia, and the like, which are difficult to determine in many cases.

In order to improve this situation and increase the positive diagnosis rate of TIA, there is a need for an index that can be objectively determined using biomarkers.

Recent advances in diagnostic techniques such as MRI imaging, ultrasonography, and the like have made it possible to investigate the mechanism in detail, and TIA, like cerebral infarction, is now being investigated to clarify the mechanism, such as cardiogenic, atherothrombotic, lacunar, and other causes, but in the case of TIA without infarct images, it is even more difficult to make a clear differential diagnosis of the cause than in cerebral infarction.

C-type lectin-like receptor 2 (CLEC2) was identified on platelets as a receptor for the platelet-activating snake venom rhodocytin. CLEC2 is expressed almost exclusively on platelets and megakaryocytes in humans, and is therefore a platelet-specific molecule. It was reported that CLEC2 is released into the blood as soluble CLEC2 (soluble CLEC-2, hereafter abbreviated as sCLEC2) when platelets are activated (Patent literature 1, Non-patent literature 1).

In the case of thrombus formation when blood vessels are injured, it is believed that platelets are activated and aggregate to form a primary thrombus, and then the coagulation system is activated, and the soluble fibrinogen becomes an insoluble fibrin network that covers the primary thrombus and forms a strong secondary thrombus. In an actual hemostatic thrombus, the platelet system and the coagulation system are activated by each other, and a hemostatic thrombus formed by platelets, fibrin, and erythrocytes entrapped by platelets and fibrin is observed.

Since the concentration of sCLEC2 in blood increases with platelet activation, it can be shown that blood sCLEC2 concentration is associated with the formation of thrombosis, and high blood sCLEC2 concentration suggests the formation of a blood clot somewhere in the body (Patent literatures 1 to 3).

### CITATION LIST

### PATENT LITERATURE

[Patent literature 1] JP 6078845 B
[Patent literature 2] Japanese Patent Application No. 2020-032797
[Patent literature 3] Japanese Patent Application No. 2021-003671
[Patent literature 4] Japanese Patent Application No. 2021-060870

### NON-PATENT LITERATURE

[Non-patent literature 1] F. Kazama et al., Platelets 2015; 26(8): 711-719
[Non-patent literature 2] Y. Yamashita et al., Thrombosis Research 178(2019) 54-58
[Non-patent literature 3] X. Zhang et al., Stroke.2019; 50: 45-52.

### SUMMARY OF INVENTION

### TECHNICAL PROBLEM

Cerebral infarction is a disease with a high mortality rate, and is also a serious disease that often leaves behind sequelae even if death is avoided. As mentioned above, about 15% of patients with TIA are known to have cerebral infarction, so it is extremely important to diagnose early and provide preventive treatment. If TIA is diagnosed, appropriate antithrombotic therapy can significantly reduce the probability of subsequent cerebral infarction. In addition, patients diagnosed with TIA can be monitored by a physician for, for example, one week after the onset of TIA, and if cerebral infarction does occur, it can be treated immediately with thrombolytic therapy to significantly improve its sequelae. The sooner cerebral infarction is treated, the better, because it is a race against time.

The diagnosis of TIA is made by interviewing clinical symptoms and evaluating the patient's risk. The main symptoms are paralysis of limbs on one side (motor impairment), numbness on one side (sensory impairment), slurred speech (speech impairment), difficulty seeing on one side (visual impairment), difficulty seeing objects on one side (visual field impairment), and the like. These symptoms disappear within a few minutes to approximately an hour at most. There is no other way to diagnose them by doctor's questions. However, since severity of the symptoms ranges from severe to weak, patients do not always accurately communicate their symptoms to their doctors.

To compensate for the difficulty of the interview, a risk evaluation of the patient is also widely used. The ABCD2 score is a typical example, which is an evaluation system that scores Age, Blood Pressure, Clinical Feature, Diabetes, and Duration of Symptoms. This is an evaluation of the risk of subsequent cerebral infarction, not a diagnosis of TIA itself. As described above, there is no objective imaging test or blood test that can complement the clinical symptom interview in diagnosing TIA.

In addition, as mentioned above, in acute cerebral vascular disease such as cerebral infarction and TIA, it is very important to distinguish and diagnose whether the cause is cardiogenic or non-cardiogenic, but the judgment is not always easy. Currently, the diagnosis of whether cerebral infarction is cardiogenic or non-cardiogenic is made comprehensively based on the results of imaging tests, electrocardiogram, echocardiography, and blood tests such as lipids, blood glucose, heart failure markers, and the like. Biomarkers such as D-dimer, soluble fibrin, and the like have been proposed to support thrombological diagnosis, but they are not accurate enough to distinguish cardiogenicity from non-cardiogenicity and have not yet been used in clinical practice. In TIA, the diagnosis of cardiogenic or non-cardiogenic is even more difficult, since the patients should have no infarction on imaging tests.

Therefore, problems of the present invention are to provide a convenient, rapid, and objective blood test-based risk evaluation method for the diagnosis of TIA that can complement the clinical symptom interview, and to provide a convenient, rapid, and objective blood test-based risk evaluation method for the differential diagnosis of cardiogenic and non-cardiogenic acute cerebral vascular diseases such as cerebral infarction and TIA that can complement imaging, electrocardiogram, and conventional blood tests.

### SOLUTION TO PROBLEM

The inventors have conducted intensive studies to solve the above-mentioned problem. As a result, we found that the blood sCLEC2 concentration in TIA patients was significantly elevated compared to that in normal subjects. We found that sCLEC2 can be elevated not only in cerebral infarction, in which there is an infarction and thrombus, but also in TIA, in which there is no infarction and no thrombus. This is consistent with the fact that patients with TIA have a high probability of having cerebral infarction in the near future. In other words, the high sCLEC2 value indicates that platelets are activated and that a thrombus of a certain size is likely to form in the near future, clogging the blood vessel and causing infarction, even though there is no infarction and no thrombus is present.

In addition, an appropriate threshold, i.e., a cutoff value, is needed to determine the risk in TIA patients using the sCLEC2 concentration. We found that the cutoff value of TIA for sCLEC2 concentration in blood should be between 66 pg/mL and 148 pg/mL.

In patients with TIA, since the infarct imaging cannot be used to determine cardiogenicity or non-cardiogenicity, blood tests are very useful to assist in the determination of cardiogenicity or non-cardiogenicity.

Furthermore, we measured sCLEC2 in the blood of patients with cerebral infarction and found that sCLEC2 measured values were significantly higher in non-cardiogenic patients than in cardiogenic patients. Based on this finding, we simultaneously measured D-dimer, which is a biomarker for the presence of a fibrin thrombus, and calculated the ratio of sCLEC2 to D-dimer (sCLEC2/D-dimer ratio). We found that the sCLEC2/D-dimer ratio in non-cardiogenic patients showed a higher value with an even larger significant difference than in cardiogenic patients. In other words, since sCLEC2 indicates platelet activity and D-dimer indicates the presence of fibrin thrombus, the ratio of sCLEC2 to D-dimer can be used to distinguish between noncardiogenic patients with platelet predominance who should be treated with antiplatelet agents and cardiogenic patients with fibrin predominance who should be treated with anticoagulants, providing a more accurate method to assist in diagnosis in addition of conventional methods.

Based on the above, we have completed the present invention as a method for evaluating the risk of acute cerebral vascular disease using soluble CLEC2.

The present invention provides the following:
[1] A method for evaluating a risk of acute cerebral vascular disease, said method comprising: measuring a concentration of soluble CLEC2 in blood collected from a patient suspected of having acute cerebral vascular disease or a patient diagnosed with acute cerebral vascular disease.
[2] The method of [1] for evaluating the risk of acute cerebral vascular disease in a patient suspected of having acute cerebral vascular disease or a patient diagnosed with acute cerebral vascular disease, said method comprising:
   (1) providing a blood sample derived from the patient;
   (2) determining the concentration of soluble CLEC2 in the sample; and
   (3) correlating the soluble CLEC2 concentration with a presence or absence of acute cerebral vascular disease in the patient, likelihood of outcome, or whether the acute cerebral vascular disease is cardiogenic or non-cardiogenic.
[3] The method of [2] for evaluating the risk of acute cerebral vascular disease in a patient suspected of having acute cerebral vascular disease or a patient diagnosed with acute cerebral vascular disease, said step of correlating the soluble CLEC2 concentration with a presence or absence of acute cerebral vascular disease in the patient, likelihood of outcome, or whether the acute cerebral vascular disease is cardiogenic or non-cardiogenic comprising:
   evaluating whether the patient is at risk based on a change in the soluble CLEC2 concentration.
[4] The method of [2] or [3] for evaluating the risk of acute cerebral vascular disease in a patient suspected of having acute cerebral vascular disease or a patient diagnosed with acute cerebral vascular disease,
   wherein the acute cerebral vascular disease is transient ischemic attack, and
   wherein, in the step of correlating the soluble CLEC2 concentration with the transient ischemic attack, a cutoff value of the soluble CLEC2 concentration is 66 to 148 pg/mL.
[5] The method of [1] for evaluating the risk of acute cerebral vascular disease in a patient suspected of having acute cerebral vascular disease or a patient diagnosed with acute cerebral vascular disease, said method comprising:
   (1) providing a blood sample derived from the patient;
   (2) determining the concentration of soluble CLEC2 in the sample;
   (3) determining platelet counts in the sample;
   (4) dividing the soluble CLEC2 concentration by the platelet counts; and
   (5) correlating the value obtained by dividing the soluble CLEC2 concentration by the platelet counts, with a presence or absence of acute cerebral vascular disease in the patient, the likelihood of outcome, or whether the acute cerebral vascular disease is cardiogenic or non-cardiogenic.
[6] The method of [5] for evaluating the risk of acute cerebral vascular disease in a patient suspected of having acute cerebral vascular disease or a patient diagnosed with acute cerebral vascular disease,
   wherein the acute cerebral vascular disease is transient ischemic attack, and
   wherein, in the step of correlating the value obtained by dividing the soluble CLEC2 concentration by the platelet counts with the transient ischemic attack, a cutoff value of the value obtained by dividing the soluble CLEC2 concentration by the platelet counts is 0.55 to 0.7.
[7] The method of any one of [1] to [4] for evaluating the risk of acute cerebral vascular disease in a patient suspected of having acute cerebral vascular disease or a patient diagnosed with acute cerebral vascular disease,
   wherein a material to judge whether the acute cerebral vascular disease is cardiogenic or non-cardiogenic is provided.
[8] The method of [1] for evaluating the risk of acute cerebral vascular disease in a patient suspected of having acute cerebral vascular disease or a patient diagnosed with acute cerebral vascular disease, said method comprising:
   (1) providing a blood sample derived from the patient;
   (2) determining the concentration of soluble CLEC2 in the sample;
   (3) determining a coagulation-fibrinolysis marker in the sample;
   (4) dividing the soluble CLEC2 concentration by the coagulation-fibrinolysis marker; and
   (5) correlating the value obtained by dividing the soluble CLEC2 concentration by the coagulation-fibrinolysis marker, with a presence or absence of acute cerebral vascular disease in the patient, the likelihood of outcome, or whether the acute cerebral vascular disease is cardiogenic or non-cardiogenic.
[9] The method of [8] for evaluating the risk of acute cerebral vascular disease in a patient suspected of having acute cerebral vascular disease or a patient diagnosed with acute cerebral vascular disease, wherein the coagulation-fibrinolysis marker is D-dimer.
[10] The method of any one of [1] to [9] for evaluating the risk of acute cerebral vascular disease in a patient suspected of having acute cerebral vascular disease or a patient diagnosed with acute cerebral vascular disease,
   wherein the sample derived from the patient is collected from the patient within 48 hours after the onset of symptoms of acute cerebral vascular disease in the providing step.
[11] The method of any one of [1] to [10], wherein the step of determining the concentration of the soluble CLEC2 is performed by highly sensitive immunoassay, such as chemiluminescence immunoassay, electrochemiluminescence immunoassay, or fluorescence immunoassay.

Furthermore, the present invention includes:
- a method for evaluating a risk of acute cerebral vascular disease, wherein a concentration of soluble CLEC2 (or a value obtained by dividing the soluble CLEC2 concentration by platelet counts, or a value obtained by dividing the soluble CLEC2 concentration by a coagulation-fibrinolysis marker) in a sample is measured (or determined);
- a method for assisting in a risk evaluation of acute cerebral vascular disease, wherein a concentration of soluble CLEC2 (or a value obtained by dividing the soluble CLEC2 concentration by platelet counts, or a value obtained by dividing the soluble CLEC2 concentration by a coagulation-fibrinolysis marker) in a sample is measured (or determined);
- a method for measuring (or determining) a concentration of soluble CLEC2 (or a value obtained by dividing the soluble CLEC2 concentration by platelet counts, or a value obtained by dividing the soluble CLEC2 concentration by a coagulation-fibrinolysis marker) in a sample for a risk evaluation of acute cerebral vascular disease;
- an in vitro method for evaluating a risk of acute cerebral vascular disease, characterized by measuring (or determining) a concentration of soluble CLEC2 (or a value obtained by dividing the soluble CLEC2 concentration by platelet counts, or a value obtained by dividing the soluble CLEC2 concentration by a coagulation-fibrinolysis marker) in a sample;
- use of an antibody capable of detecting a concentration of soluble CLEC2 in the manufacture of a kit for a risk evaluation of acute cerebral vascular disease; and
- a method for measuring (or determining) a concentration of soluble CLEC2 (or a value obtained by dividing the soluble CLEC2 concentration by platelet counts, or a value obtained by dividing the soluble CLEC2 concentration by a coagulation-fibrinolysis marker) in a sample to provide information necessary for a risk evaluation of acute cerebral vascular disease.

### ADVANTAGEOUS EFFECTS OF INVENTION

The method of the present invention, measuring the concentration of sCLEC2 present in the blood of a patient with acute cerebral vascular disease, or using the ratio of sCLEC2/D-dimer, enables a convenient, rapid, and objective risk evaluation of acute cerebral vascular disease. Furthermore, it is expected to contribute to improving the accuracy of determining the efficacy of treatment in monitoring prophylactic treatment of cerebral infarction or TIA after the diagnosis of TIA.

### BRIEF DESCRIPTION OF THE DRAWINGS

[Fig. 1] Figure 1 is a standard curve prepared using a human sCLEC2 protein as a standard.
[Fig. 2] Figure 2 is a graph in which the concentrations of sCLEC2 in plasma were compared between TIA patients and normal subjects.
[Fig. 3] Figure 3 is an ROC curve (Receiver Operating Characteristic Curve) in the diagnosis of TIA by measuring the concentrations of sCLEC2 in blood.
[Fig. 4] Figure 4 is a graph in which the concentrations of sCLEC2 in normal subjects and patients with cerebral infarction were measured and the concentrations of sCLEC2 were compared among three groups of normal subjects, patients with cardiogenic cerebral infarction, and patients with non-cardiogenic cerebral infarction.
[Fig. 5] Figure 5 is an ROC curve in the differential diagnosis between cardiogenic cerebral infarction and non-cardiogenic cerebral infarction using the concentrations of sCLEC2 in patients with cerebral infarction.
[Fig. 6] Figure 6 is an ROC curve in the differential diagnosis between cardiogenic cerebral infarction and non-cardiogenic cerebral infarction using the concentrations of D-dimer in patients with cerebral infarction.
[Fig. 7] Figure 7 is an ROC curve in the differential diagnosis between cardiogenic cerebral infarction and non-cardiogenic cerebral infarction using the sCLEC2/D-dimer ratio in patients with cerebral infarction.

### DESCRIPTION OF EMBODIMENTS

Hereinafter, the embodiments of the present invention will be explained in detail, but the embodiments of the use are not limited thereto.

The term "CLEC2" as used herein is a platelet-activating receptor belonging to a C-type lectin family, which is usually present on the membrane of platelets and is released into the blood upon platelet activation. The term "soluble CLEC2 (sCLEC2)" as used herein means CLEC2 or CLEC2-derived molecules that are released from such platelets and detected in blood (or a buffer if incubated in the buffer).

sCLEC2 includes proteins with molecular weights of approximately 40 kDa, approximately 32 kDa, approximately 25 kDa, and the like in SDS-polyacrylamide gel electrophoresis (SDS-PAGE) under reducing conditions (Non-patent literature 1). The proteins with molecular weights of approximately 40 kDa and approximately 32 kDa are present on the membrane surface of platelets, and it is presumed to be released in the form of being included in microparticles produced upon platelet activation. These are thought to have sugar chains attached to them. On the other hand, the protein with a molecular weight of approximately 25 kDa are thought to be cleaved by proteases and released from platelets upon platelet activation. In the present invention, the amount of sCLEC2 as described above is measured. sCLEC2 can be detected together as proteins with molecular weights of approximately 40 kDa, approximately 32 kDa, and approximately 25 kDa, or it can be detected only as a protein with a molecular weight of approximately 25 kDa.

As the concentration of sCLEC2 used in the present invention, the concentration of sCLEC2 may be used alone, or may be used in combination with other biomarkers. Preferably, for example, in the diagnosis of TIA, the value obtained by dividing the sCLEC2 concentration by platelet counts (hereinafter sometimes referred to as C2PAC index) may be used. In the present specification, unless otherwise specified, the concentration of sCLEC2 is interpreted to include both the case of using the sCLEC2 concentration and the case of dividing the sCLEC2 concentration by platelet counts. Furthermore, in the differential diagnosis between cardiogenic cerebral infarction and non-cardiogenic cerebral infarction, not only the sCLEC2 concentration, but also the sCLEC2/D-dimer ratio obtained by dividing the sCLEC2 concentration by D-dimer, can be used.

Samples for the measurement are preferably derived from humans, but samples derived from non-human animals may be used for the purpose of understanding the clinical conditions or the like of experimental animals. Such experimental animals are not limited, but include, for example, a guinea pig, a rat, a mouse, a dog, and the like.

A method for detecting the presence of sCLEC2 is not limited, but immunological methods using an antibody that recognizes sCLEC2 (hereinafter sometimes referred to as "anti-sCLEC2 antibody") are preferred. As the methods in which the protein is immunologically detected, any of the following methods which are known and commonly used can be used: for example, immunoassays using labeled antibodies, such as enzyme-linked immunoassay (ELISA), chemiluminescence immunoassay, electrochemiluminescence immunoassay, fluorescent immunoassay, radioimmunoassay, immunochromatography, and the like; or Western blotting, latex agglutination, immunoturbidimetric method, and the like. However, a simple immunoassay may not be sensitive enough to measure the sCLEC2 concentration in normal subjects, so an assay that is sensitive enough to measure blood sCLEC2 of normal subjects should be used. For this purpose, highly sensitive assays are desirable, and chemiluminescence immunoassay, electrochemiluminescence immunoassay, fluorescence immunoassay, and the like are most preferably used.

A sample is collected from a subject (in particular, a patient), for example, with a blood collection tube for plasma collection. Citrate blood collection tubes with low residual platelets are usually suitable, but heparin blood collection tubes or EDTA blood collection tubes can also be used. Although EDTA blood collection tubes are used for measuring platelet counts, separate blood collection tubes may be used for simultaneous blood collection. The sCLEC2 concentration in plasma is measured by using centrifuged plasma, for example, at 2000g for approximately 20 minutes, but conditions for centrifugation are not limited to this, and whole blood may also be used. The following explanation is based on, but not limited to, the measurement of the sCLEC2 concentration in plasma. It is preferable to collect blood from a patient no later than 48 hours after the onset of symptoms, but blood can be used in the present invention even if 48 hours have elapsed. It is more preferably within 24 hours, still more preferably within 18 hours, and most preferably within 12 hours.

The term "acute cerebral vascular disease" as used herein includes, but not limited to, ischemic stroke, such as cerebral infarction, and TIA.

TIA means transient cerebral infarction-like symptoms with no infarction confirmed by imaging tests. Cerebral infarction-like symptoms include, but are not limited to, for example, paralysis of limbs on one side (motor impairment), numbness on one side (sensory impairment), slurred speech (speech impairment), difficulty seeing on one side (visual impairment), difficulty seeing objects on one side (visual field impairment), and the like. Since these symptoms disappear within a few minutes to approximately an hour at most, there is no other way to diagnose them by doctor's questions. The severity of the symptoms, which ranges from severe to weak, does not matter. Patients with cerebral infarction are those who, in addition to the clinical symptoms above, have an infarct image confirmed in their brain by MRI, CT, or the like.

Since cerebral infarction is a condition in which an artery in the brain is occluded (infarction) and it is clear that a thrombus is present in this case, it is reasonable to expect that the blood sCLEC2 level, a marker of platelet activation, is elevated. Furthermore, since it is pathologically clear that disseminated intravascular coagulation syndrome (DIC), thrombotic microangiopathy (TMA), and deep vein thrombosis are also pathological conditions in which a thrombus is seen, it is not surprising that sCLEC2 is elevated. However, when it was thought that markers related to thrombosis (for example, D-dimer is well known) were not so elevated because the presence of a thrombus was not confirmed when infarction was not confirmed, as in TIA, it was surprising that the concentration of sCLEC2, which is suggested to be used as a marker for thrombosis, was elevated and can be used as a blood biomarker for risk evaluation in TIA.

As a method of conducting risk evaluation, when the sCLEC2 concentration of a patient with suspected TIA is higher than that of normal subjects or a non-thrombotic disease group, it can be judged that the patient is likely to have TIA. Based on such a comparison, the sCLEC2 concentration can be used as a risk prediction of cerebral infarction onset by comparing sCLEC2 concentrations before and after prophylactic treatment for a TIA patient.

For the correlation between the measured sCLEC2 concentration in a patient-derived sample and the possibility of TIA, a threshold can be appropriately set and used based on the comparison of sCLEC2 concentrations in patient-derived samples and those in normal subject-derived samples. For the risk prediction of cerebral infarction onset, TIA can be evaluated when a significant change in sCLEC2 concentration is detected from the temporal record of sCLEC2 concentrations measured before the onset in the same patient.

Specifically, when the blood sCLEC2 concentration in a patient who describes symptoms of suspected TIA is measured and the value is higher than those of normal patients or the like, it can be judged that there is a high probability of TIA, and then, antiplatelet agents such as aspirin or anticoagulants such as warfarin can be administered prophylactically, depending on risk evaluation after further examination. Furthermore, the sCLEC2 concentration is measured after taking antiplatelet agents or anticoagulants for treatment after being diagnosed with TIA, and if the value is high, the drug is changed to a different type of drugs or a different type of drugs is additionally administered. These contribute to the consideration for determining treatment policy.

As original data, statistical processing data, or the like to calculate the threshold value (cutoff value) for judgment, the correlation between the sCLEC2 concentration in plasma and the degree of platelet activation or various diseases may be used.

For example, as a method of calculating the cutoff value, the analysis to create a ROC curve (Receiver Operating Characteristic Curve) from the measured sCLEC2 values in plasma can be performed, and the concentration that shows 80% or more in both sensitivity and specificity for diagnosis can be used as the cutoff value.

In the method of the present invention, the cutoff value of TIA by sCLEC2 is preferable for any value between 66 pg/mL to 148 pg/mL. As a more preferable embodiment, the same analysis can be performed when the C2PAC index, which is a value obtained by dividing the sCLEC2 concentration by the platelet counts, is used to calculate the cutoff value. The cutoff value can be set to 0.7 when the C2PAC index is used.

sCLEC2 is released into the blood upon platelet activation. Conventional platelet activation markers, such as platelet factor 4 (PF4) and β-thromboglobulin (β-TG), have problems, because the physical stimulation by blood collection causes nonspecific release. However, sCLEC2 is released by a signal transduction-dependent mechanism that triggers platelet activation, and may be a more accurate marker for platelet activation in vivo. In addition, CLEC2 may be a platelet-specific marker with few false positives because its expression is almost limited to the platelet and megakaryocyte system in humans. Therefore, measurement of sCLEC2 enables early diagnosis of platelet activation status, and can be used for the diagnosis of TIA.

The value obtained by dividing the measured sCLEC2 concentration by platelet counts may be used for risk evaluation. If the sCLEC2 concentration decreases with treatment, platelet activation is suppressed, but if the sCLEC2 concentration remains high or increases, change, addition, dosage increase, or the like of antithrombotic agents may be considered. In the present specification, the value obtained by dividing the sCLEC2 concentration by platelet counts is referred to as C2PAC index. In the diagnosis of TIA, all the items described as sCLEC2 concentration can be performed by replacing the sCLEC2 concentration with the C2PAC index, even if they are not explicitly described as C2PAC index.

When the sCLEC2 concentration is divided by platelet counts for the diagnosis of TIA, the platelet counts are usually measured using an automated hemocytometer (blood counter), but can also be counted using a hemocytometer plate and a microscope.

The sCLEC2 concentration in plasma is expressed in, for example, pg/mL, and the platelet counts in blood are expressed in, for example, 1,000 platelets/mm³, and it is preferable to use the C2PAC index calculated using these values. The concentration of sCLEC2 used herein can be expressed in ng/mL, ng/L, or any other arbitrary unit, and the platelet counts can be expressed in 10,000 platelets/mm³ or any other arbitrary unit, but uniform units should be used for comparison. By using various units, sCLEC2 concentration/platelet counts can take various values, but they are essentially the same concept.

It can be expected that the calculation of the ratio is often performed using measured values from clinical laboratory equipment that measures the sCLEC2 concentration and measured values from a blood count meter that measures platelet counts. Although it is preferable in daily practice to perform this calculation automatically on a hospital laboratory system, a hospital system, an electronic medical record system, or the like that is connected to both analyzers, it is also possible to construct a system that connects the data of the two analyzers, or to construct a machine that can simultaneously measure the sCLEC2 concentration and platelet counts. Furthermore, the ratio may also be computed manually using both data.

Furthermore, the correlation between the sCLEC2 concentration in plasma and the degree of platelet activation or various diseases may be used, for example, as a threshold for judgment, or as original data or statistical processing data to calculate the threshold for judgment.

The diagnosis of acute cerebral vascular disease by dividing the sCLEC2 concentration in plasma by platelet counts in blood and calculating the amount of sCLEC2 released per platelet as an index is preferable because it is possible to evaluate the degree of platelet activation without depending on the number of platelets in blood. Specifically, for example, the sCLEC2 concentration in plasma is expressed in pg/mL (A), platelet counts in blood is expressed in 1,000 platelets/mm³ (B), and the number obtained by dividing A by B can be used as an index of platelet activation.

This invention does not preclude a use in the diagnosis of cerebral infarction. The diagnosis of cerebral infarction is based on a comprehensive judgment, taking into account the symptoms such as paralysis of limbs on one side (motor impairment), numbness on one side (sensory impairment), slurred speech (speech impairment), difficulty seeing on one side (visual impairment), difficulty seeing objects on one side (visual field impairment), and the like, and the confirmation of infarction by imaging tests such as MRI. In patients with suspected cerebral infarction, measurement of the sCLEC2 concentration in blood by the present invention is preferred because it can assist in a quick and convenient diagnosis of cerebral infarction. In addition, in clinical practice, it is important to be able to objectively judge not only cerebral vascular diseases including cerebral infarction but also whether it is cardiogenic or non-cardiogenic by a quick and convenient method, and the present invention can meet this demand.

In order to make a differential diagnosis of cardiogenic cerebral infarction or non-cardiogenic cerebral infarction, for example, the sCLEC2 concentration in blood are measured in patients diagnosed with acute cerebral vascular disease. In this case, when the sCLEC2 concentration is found to be significantly elevated, and if it is higher than a predetermined threshold, it can be judged the probability of non-cardiogenic cerebral infarction is high, and if it is lower than the threshold, it can be judged that the probability of cardiogenic cerebral infarction is high.

In addition, the sCLEC2 concentration in combination with other coagulation-fibrinolysis markers can be used for a differential diagnosis of cardiogenic cerebral infarction or non-cardiogenic cerebral infarction. For example, the sCLEC2 concentration and the D-dimer concentration in blood are measured in a patient diagnosed with acute cerebral vascular disease. The measurements are preferably performed on samples collected at the same time, but the measurements may also be performed on samples collected separately. Those skilled in the art can perform the blood sampling by judging the degree of influence appropriately, even if there is a time difference between the time of blood sampling when each concentration is measured.

It can be judged that if the sCLEC2/D-dimer ratio, which is the sCLEC2 concentration divided by the D-dimer concentration, is higher than a certain threshold, the probability of non-cardiogenicity is high, and it can be judged that if the sCLEC2/D-dimer ratio is lower than the threshold, the probability of cardiogenicity is high. When the sCLEC2 concentration divided by the D-dimer concentration is used to calculate the sCLEC2/D-dimer ratio, D-dimer can be expressed in various units such as µg/mL, ng/L, or the like, but a uniform concentration unit should be used for comparison. The general expression of µg/mL is preferable, but is not limited to this.

As coagulation-fibrinolysis markers other than D-dimer, which can express the presence of fibrin thrombus and fibrin formation,
FDP (fibrin/fibrinogen degradation products), soluble fibrin (activation marker of thrombin), fibrin monomer, thrombin-antithrombin complex (TAT), and prothrombin fragment F1+2 can also be used to judge cardiogenicity/noncardiogenicity by taking a ratio to sCLEC2.

It is easy to diagnose cardiogenic cerebral infarction when a relatively large infarct is seen on images such as MRI or the like, and atrial fibrillation, arrhythmia, or the like is present. In some cases, typical atherosclerotic cerebral infarction can be easily estimated, such as moderate infarct size and atherosclerotic stenosis in the carotid artery, or stenosis in the artery near the infarction. In cases where neither of these is typical, it is often difficult to determine the judge. However, since the treatment depends critically on the determination of the diagnosis, in actual clinical practice, the diagnosis is made comprehensively by clinical symptoms, medical history, electrocardiogram, blood tests, and the like, in addition to imaging tests such as MRI, MRA, CT, echocardiography, and the like. Additionally, thrombological information, such as whether the infarcted thrombus is fibrin-predominant or platelet-predominant, is very useful for diagnosis. In the case of cerebral infarction, the start of treatment is a matter of time, but in many cases, the time elapsed before the patient is seen in an outpatient clinic, such as an emergency room, is unknown. The use of such a risk evaluation method is desirable because it enables rapid selection of appropriate treatment if a differential diagnosis of cardiogenic or non-cardiogenic cerebral infarction can be made by a simple method such as the present invention.

As treatment for acute cerebral infarction, anticoagulants and antiplatelet agents are known as intravenous therapies, but even if they are the same anticoagulants, they may have different indications. For example, in the anticoagulants argatroban and heparin, argatroban is indicated for non-cardiogenic cerebral infarction, whereas heparin is indicated for cardiogenic cerebral infarction. In addition, the applicable therapeutic agents may differ depending on the time elapsed since the onset of cerebral infarction. Anticoagulant (argatroban) is indicated if the infarct size is 1.5 cm or larger within 2 days of onset, but not if it is not large or if 3 days or more have passed. Thus, in deciding on a treatment plan, the implementation of the present invention is preferred because it not only diagnoses cerebral infarction but also quickly differentiates between cardiogenic and non-cardiogenic infarction.

In the past, there were some proposals for the use of drugs such as edaravone, which has a completely different mechanism of action from anticoagulants and antiplatelet agents and is indicated for both cardiogenic and non-cardiogenic patients, and is thought to reduce exacerbation of cerebral infarction by removing free radicals. However, there are cases in which the treatment strategy differs from country to country, such as the lack of mention of edaravone in recent treatment guidelines in the United States. In the above situation, it is preferable to implement the present invention not only to diagnose cerebral infarction but also to quickly differentiate between cardiogenic and non-cardiogenic infarction in order to respond to the constantly evolving treatment policy decisions.

In the acute phase of cerebral infarction, in some cases anticoagulants and antiplatelet agents are first used in combination, and the next course of treatment is decided after about one to two weeks of monitoring the effects of these drugs. In this case, aspirin or warfarin is often selected as the next oral drug. However, there is no difficulty in judgment as to which drug to choose if imaging tests show lacunar cerebral infarction, atherosclerotic cerebral infarction, or cardiogenic cerebral infarction clearly, but even specialists sometimes differ in their judgment, making it difficult to decide. If the diagnosis cannot be made based on imaging tests alone, a comprehensive judgment is made based on the results of 24-hour electrocardiogram, implantable electrocardiogram, carotid echocardiogram, transthoracic echocardiogram, and transesophageal echocardiogram. Since there are many cases in which it is difficult to determine whether the cerebral infarction is cardiogenic or non-cardiogenic even after such tests, it is extremely effective to be able to determine whether the cerebral infarction is cardiogenic or non-cardiogenic with high accuracy and simplicity by using the biomarker-based test of the present invention in such cases.

The use in differential diagnosis of cerebral infarction as cardiogenic or non-cardiogenic is described above, but it can also be used in differential diagnosis of TIA as cardiogenic or non-cardiogenic. The same method can be used in the differential diagnosis of cardiogenic and non-cardiogenic TIA as in the differential diagnosis of cardiogenic or non-cardiogenic cerebral infarction. In TIA, since the infarct image by MRI or other imaging methods is not seen, this type of differential diagnosis can be made, which is even more preferable.

### EXAMPLES

The present invention will now be further illustrated by, but is by no means limited to, the following Examples.

### «Example 1: Measurement of sCLEC2 in human plasma»

In this Example, plasma sCLEC2 concentrations were measured as follows.

### (Preparation of reagent for measurement and preparation of test samples)

· Sample diluent liquid: 0.1 mol/L HEPES buffer (pH 7.5) containing a preservative was used with a combination of 2% sodium octanoate and 0.5% n-octyl-β-D-glucoside (OG) to prepare the sample diluent liquid.

The antibodies in the reagent were prepared as follows, using antibodies described in Examples of Japanese Patent No. 6078845.
· First antibody solution: A mouse monoclonal antibody (11D5) recognizing sCLEC2 was carried on magnetic latex particles (JSR Corporation) and dispersed in 0.01 mol/L MES buffer (pH 6.0) containing a preservative.
· Second antibody solution: Another mouse monoclonal antibody (11E6) recognizing sCLEC2 was labeled with alkaline phosphatase (ALP) by a maleimide method and dispersed in 0.01 mol/L MES buffer (pH 6.5) containing a preservative.
· Luminescent substrate solution: Disodium 2-chloro-5-(4-methoxyspiro{1,2-dioxetane-3,2'-(5'-chloro)-tricyclo[3.3.1.13,7]decane}-4-yl)-1-phenyl phosphate (CDP-Star (registered trademark): Applied Biosystems) was used.
· B/F washing buffer: A buffer solution containing 0.1 mol/L citric acid (pH 6.5), 0.15 mol/L NaCl, and 0.1% TritonX-100 was used.
· Test samples: A recombinant human soluble CLEC2 (hsCLEC2)protein diluted with a buffer solution (0.025 mol/L HEPES, 0.14 mol/L NaCl, 0.1% sodium octanoate, and 0.3% BSA) was used as test sample 1, and the same diluted with citrate plasma was used as test sample 2.

### (Measurement by measurement reagent)

An automated clinical testing system STACIA (registered trademark, LSI Medience Corporation) was used for the measurement.

STACIA-dedicated bottles were filled with the prepared sample diluent liquid, the first antibody solution (magnetic latex reagent), and the second antibody solution (enzyme-labeled antibody reagent), respectively, and placed in the apparatus. The measurement was performed according to the operation method of the apparatus.

Specifically, 40 µL of the sample diluent liquid was added to 10 µL of the sample and warmed at 37°C for several minutes, and then 25 µL of the first antibody solution (magnetic latex reagent) was added and warmed at 37°C for several minutes. Then, B/F separation was performed, 50 µL of the second antibody solution (enzyme-labeled antibody reagent) was added, and the mixture was warmed at 37°C for several minutes. B/F separation was performed again, 100 µL of the luminescent substrate solution was added, and the signal intensity (counts) was measured after several minutes of reaction at 37°C.

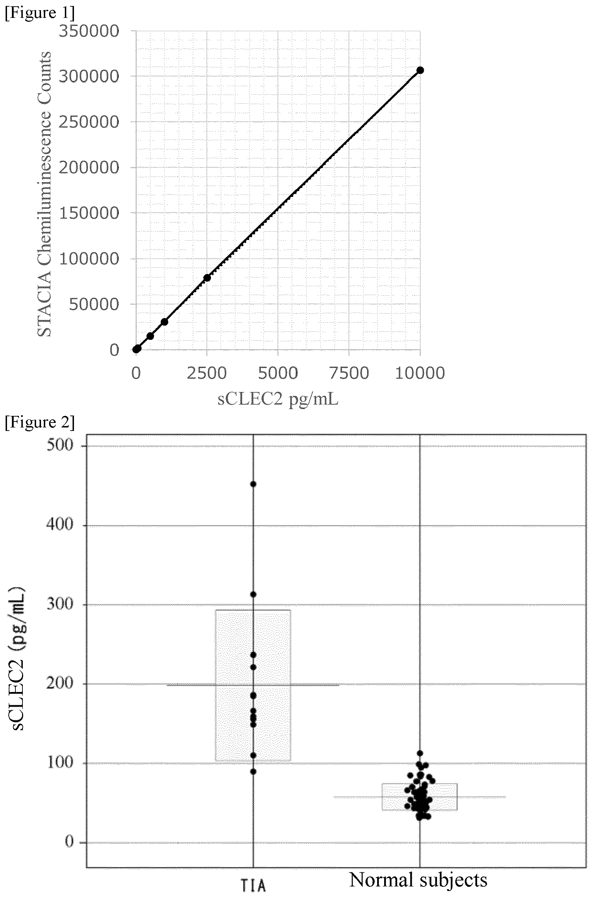

Figure 1 shows a standard curve prepared using the hsCLEC2 protein as a standard.

### <<Example 2: Measurement of sCLEC2 in plasma samples from TIA patients and normal subjects>>

Plasma sCLEC2 concentrations, platelet counts, and the sCLEC2/platelet ratios (C2PAC index) were measured according to the method of Example 1, using citrate plasma obtained from patients diagnosed with TIA based on doctor's questions about clinical symptoms and without new infarct image on MRI, and normal subjects (Table 1 and Table 2).

**[Table 1]**

| sCLEC2 measured values in TIA patients | | | |
|---|---|---|---|
| TIA case | sCLEC2 (pg/mL) | Platelet counts (×1000/µL) | C2PAC index |
| 1 | 89.6 | 139 | 0.64 |
| 2 | 236.6 | 222 | 1.07 |
| 3 | 184.6 | 179 | 1.03 |
| 4 | 221.2 | 324 | 0.68 |
| 5 | 186.1 | 217 | 0.86 |
| 6 | 155.4 | 270 | 0.58 |
| 7 | 148.5 | 185 | 0.80 |
| 8 | 110.1 | 131 | 0.84 |
| 9 | 313.2 | 314 | 1.00 |
| 10 | 156.0 | 160 | 0.98 |
| 11 | 159.0 | 184 | 0.86 |
| 12 | 451.8 | 191 | 2.37 |

**[Table 2]**

| sCLEC2 measured values in normal subjects | | | | | |
|---|---|---|---|---|---|
| Normal subject | sCLEC2 | Normal subject | sCLEC2 | Normal subject | sCLEC2 |
| 1 | 112.7 | 25 | 82.8 | 49 | 45.8 |
| 2 | 85.7 | 26 | 70.9 | 50 | 67.0 |
| 3 | 42.7 | 27 | 53.9 | 51 | 55.6 |
| 4 | 47.5 | 28 | 54.0 | 52 | 55.5 |
| 5 | 54.5 | 29 | 43.8 | 53 | 33.0 |
| 6 | 64.4 | 30 | 73.2 | 54 | 63.8 |
| 7 | 57.8 | 31 | 63.8 | 55 | 40.1 |
| 8 | 51.7 | 32 | 84.7 | 56 | 48.9 |
| 9 | 57.2 | 33 | 44.5 | 57 | 34.1 |
| 10 | 98.8 | 34 | 58.0 | 58 | 48.4 |
| 11 | 56.6 | 35 | 77.7 | 59 | 83.5 |
| 12 | 63.6 | 36 | 97.6 | 60 | 41.7 |
| 13 | 49.4 | 37 | 47.7 | 61 | 34.1 |
| 14 | 34.0 | 38 | 57.3 | 62 | 48.5 |
| 15 | 46.9 | 39 | 61.0 | 63 | 31.5 |
| 16 | 52.0 | 40 | 65.9 | 64 | 54.0 |
| 17 | 52.2 | 41 | 52.3 | 65 | 58.8 |
| 18 | 44.7 | 42 | 70.2 | 66 | 48.9 |
| 19 | 85.8 | 43 | 43.3 | 67 | 51.3 |
| 20 | 61.8 | 44 | 63.5 | 68 | 44.7 |
| 21 | 94.7 | 45 | 47.6 | 69 | 48.2 |
| 22 | 59.0 | 46 | 55.5 | 70 | 50.7 |
| 23 | 45.0 | 47 | 77.2 | 71 | 42.4 |
| 24 | 42.5 | 48 | 49.6 | | |

**[Table 3]**

| Statistical data of sCLEC2 values in normal subjects and TIA patients | | |
|---|---|---|
| | Normal subjects (71 cases) | TIA cases (13 cases) |
| Average | 51.9 | 198.3 |
| Standard deviation | 16.7 | 94.9 |
| Median | 54.6 | 166.0 |

Using these measured values, statistical data of sCLEC2 values for normal subjects and TIA patients were generated (Table 3). The average, standard deviation, and median values of sCLEC2 measured value (pg/mL) were 198.3, 94.9, and 166.0 in TIA cases, and 51.9, 16.7, and 54.6 in normal subjects measured separately. The sCLEC2 values in TIA patients were significantly higher than those in normal subjects (P<0.0001). Each measured value is shown in Figure 2.

The upper limit of the C2PAC index measured separately for normal subjects was 0.7.

### <<Example 3: Sensitivity and specificity of diagnosis for TIA by sCLEC2>>

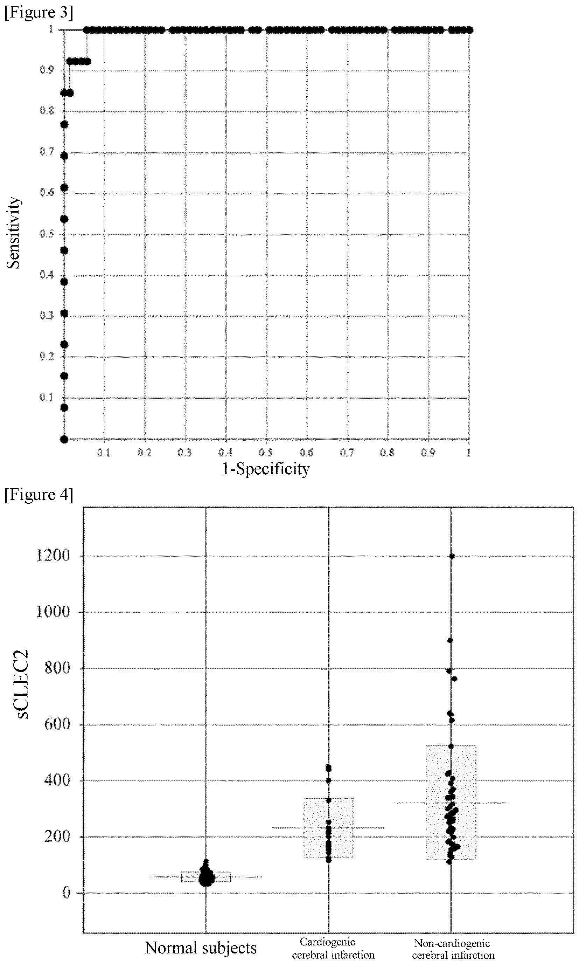

When the measured values in Example 2 were analyzed by changing the cutoff value of sCLEC2, the ROC curve (Receiver Operating Characteristic Curve) as shown in Figure 3 was obtained, and the area under the curve was 0.994, which was very good.

Table 4 shows the sensitivity and specificity of the diagnosis when the cutoff values were changed from 60 pg/mL to 150 pg/mL in this analysis. The cutoff values that showed 80% or more for both sensitivity and specificity were from 66 pg/mL to 148 pg/mL.

**[Table 4]**

| Cutoff value (pg/mL) | Sensitivity | Specificity |
|---|---|---|
| 148.5 | 76.9% | 100.0% |
| 112.7 | 84.6% | 100.0% |
| 110.1 | 84.6% | 98.6% |
| 98.8 | 92.3% | 98.6% |
| 97.6 | 92.3% | 97.2% |
| 94.7 | 92.3% | 95.8% |
| 89.6 | 92.3% | 94.4% |
| 85.8 | 100.0% | 94.4% |
| 85.7 | 100.0% | 93.0% |
| 84.7 | 100.0% | 91.5% |
| 83.5 | 100.0% | 90.1% |
| 82.8 | 100.0% | 88.7% |
| 77.7 | 100.0% | 87.3% |
| 77.2 | 100.0% | 85.9% |
| 73.2 | 100.0% | 84.5% |
| 70.9 | 100.0% | 83.1% |
| 70.2 | 100.0% | 81.7% |
| 67 | 100.0% | 80.3% |
| 65.9 | 100.0% | 78.9% |
| 64.4 | 100.0% | 77.5% |
| 63.8 | 100.0% | 76.1% |
| 63.6 | 100.0% | 73.2% |
| 63.5 | 100.0% | 71.8% |
| 61.8 | 100.0% | 70.4% |
| 61 | 100.0% | 69.0% |

When the cutoff value for the sCLEC2 concentration is set at 100 pg/mL, the diagnostic performance of sCLEC2 for TIA is: sensitivity (11/12) = 91.6%, specificity (70/71) = 98.6%, and accuracy (81/83) = 97.6% (Table 5). When the cutoff value for the C2PAC index is set at 0.55, There were 10 cases with a C2PAC index of 0.7 or higher and with a sensitivity of 83.3% in TIA patients.

In conclusion, the measurement of sCLEC2, and the sCLEC2/platelet ratio (C2PAC index) can provide convenient, accurate, and objective data for the diagnosis of TIA.

**[Table 5]**

| | TIA | Normal subjects | Total |
|---|---|---|---|
| sCLEC2 > 100 pg/mL | 12 | 1 | 13 |
| sCLEC2 < 100 pg/mL | 1 | 70 | 71 |
| Total | 13 | 71 | 84 |

### <<Example 4: sCLEC2, D-dimer, and sCLEC2/D-dimer ratio in each type of cerebral infarction and TIA>>

Among patients diagnosed with cerebral infarction, blood sCLEC2 was measured in patients with cardiogenic cerebral infarction, patients with atherosclerotic cerebral infarction, and patients with lacunar infarction by the method of Example 1 (Figure 4). When a significant difference between the sCLEC2 concentration in patients with cardiogenic cerebral infarction and those in patients with non-cardiogenic was confirmed by the Mann-Whitney U test, p-value was 0.002720 (<0.05), and it was confirmed that sCLEC2 is useful in differentiating between cardiogenic cerebral infarction and non-cardiogenic cerebral infarction.

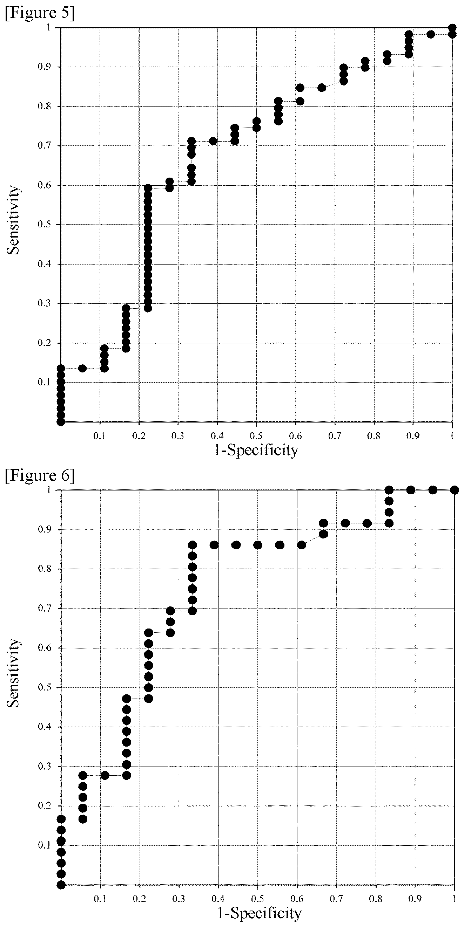

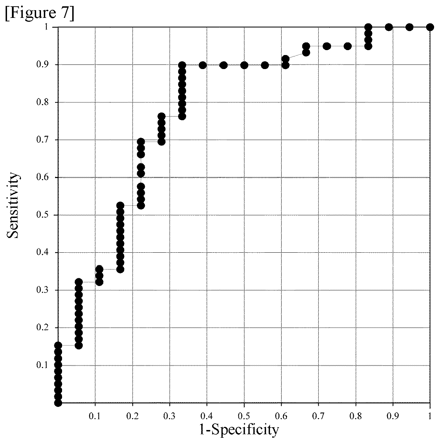

Furthermore, in order to more accurately differentiate between cardiogenic cerebral infarction and non-cardiogenic cerebral infarction, D-dimer contained in samples collected from the above cerebral infarction patients was measured using LPIA-Genesis D-Dimer (LSI Medience Corporation). An automated clinical testing system STACIA (LSI Medience Corporation) was used for the measurement. When D-dimer, sCLEC2, and the sCLEC2/D-dimer ratio was used, the diagnostic performance to differentiate between non-cardiogenic cerebral infarction (atherosclerotic cerebral infarction and lacunar infarction) and cardiogenic cerebral infarction was analyzed using an ROC curve (Figure 5 to Figure 7). The areas under the curve of D-dimer, sCLEC2, and the sCLEC2/D-dimer ratio were 0.67, 0.73, and 0.79, respectively, and the sCLEC2/D-dimer ratio showed the best diagnostic performance.

When representative cases of TIA patients suspected of being cardiogenic were similarly confirmed using sCLEC2 and the sCLEC2/D-dimer ratio, as in the case of cerebral infarction, a tendency to be lower than in patients with non-cardiogenic TIA was confirmed.

### INDUSTRIAL APPLICABILITY

As described above, the measurements of blood sCLEC2 and the ratio of sCLEC2/platelet in the present invention can be clinical tests for the diagnosis of TIA, and the sCLEC2 measuring reagent can be a clinical diagnostic reagent for TIA. Furthermore, blood sCLEC2 and the ratio of sCLEC2/D-dimer can be clinical tests for the cardiogenic/non-cardiogenic diagnosis of cerebral infarction or TIA, and the sCLEC2 measuring reagent can be a clinical diagnostic reagent for the cardiogenic/non-cardiogenic diagnosis of cerebral infarction or TIA.

## Claims

1. A method for evaluating a risk of acute cerebral vascular disease, said method comprising:
measuring a concentration of soluble CLEC2 in blood collected from a patient suspected of having acute cerebral vascular disease or a patient diagnosed with acute cerebral vascular disease.

2. The method according to claim 1 for evaluating the risk of acute cerebral vascular disease in a patient suspected of having acute cerebral vascular disease or a patient diagnosed with acute cerebral vascular disease, said method comprising:
(1) providing a blood sample derived from the patient;
(2) determining the concentration of soluble CLEC2 in the sample; and
(3) correlating the soluble CLEC2 concentration with a presence or absence of acute cerebral vascular disease in the patient, likelihood of outcome, or whether the acute cerebral vascular disease is cardiogenic or non-cardiogenic.

3. The method according to claim 2 for evaluating the risk of acute cerebral vascular disease in a patient suspected of having acute cerebral vascular disease or a patient diagnosed with acute cerebral vascular disease, said step of correlating the soluble CLEC2 concentration with a presence or absence of acute cerebral vascular disease in the patient, likelihood of outcome, or whether the acute cerebral vascular disease is cardiogenic or non-cardiogenic comprising:
evaluating whether the patient is at risk based on a change in the soluble CLEC2 concentration.

4. The method according to claim 2 or 3 for evaluating the risk of acute cerebral vascular disease in a patient suspected of having acute cerebral vascular disease or a patient diagnosed with acute cerebral vascular disease,
wherein the acute cerebral vascular disease is transient ischemic attack, and
wherein, in the step of correlating the soluble CLEC2 concentration with the transient ischemic attack, a cutoff value of the soluble CLEC2 concentration is 66 to 148 pg/mL.

5. The method according to claim 1 for evaluating the risk of acute cerebral vascular disease in a patient suspected of having acute cerebral vascular disease or a patient diagnosed with acute cerebral vascular disease, said method comprising:
(1) providing a blood sample derived from the patient;
(2) determining the concentration of soluble CLEC2 in the sample;
(3) determining platelet counts in the sample;
(4) dividing the soluble CLEC2 concentration by the platelet counts; and
(5) correlating the value obtained by dividing the soluble CLEC2 concentration by the platelet counts, with a presence or absence of acute cerebral vascular disease in the patient, the likelihood of outcome, or whether the acute cerebral vascular disease is cardiogenic or non-cardiogenic.

6. The method according to claim 5 for evaluating the risk of acute cerebral vascular disease in a patient suspected of having acute cerebral vascular disease or a patient diagnosed with acute cerebral vascular disease,
wherein the acute cerebral vascular disease is transient ischemic attack, and
wherein, in the step of correlating the value obtained by dividing the soluble CLEC2 concentration by the platelet counts with the transient ischemic attack, a cutoff value of the value obtained by dividing the soluble CLEC2 concentration by the platelet counts is 0.55 to 0.7.

7. The method according to any one of claims 1 to 4 for evaluating the risk of acute cerebral vascular disease in a patient suspected of having acute cerebral vascular disease or a patient diagnosed with acute cerebral vascular disease,
wherein a material to judge whether the acute cerebral vascular disease is cardiogenic or non-cardiogenic is provided.

8. The method according to claim 1 for evaluating the risk of acute cerebral vascular disease in a patient suspected of having acute cerebral vascular disease or a patient diagnosed with acute cerebral vascular disease, said method comprising:
(1) providing a blood sample derived from the patient;
(2) determining the concentration of soluble CLEC2 in the sample;
(3) determining a coagulation-fibrinolysis marker the sample;
(4) dividing the soluble CLEC2 concentration by the coagulation-fibrinolysis marker; and
(5) correlating the value obtained by dividing the soluble CLEC2 concentration by the coagulation-fibrinolysis marker, with a presence or absence of acute cerebral vascular disease in the patient, the likelihood of outcome, or whether the acute cerebral vascular disease is cardiogenic or non-cardiogenic.

9. The method according to claim 8 for evaluating the risk of acute cerebral vascular disease in a patient suspected of having acute cerebral vascular disease or a patient diagnosed with acute cerebral vascular disease,
wherein the coagulation-fibrinolysis marker is D-dimer.

10. The method according to any one of claims 1 to 9 for evaluating the risk of acute cerebral vascular disease in a patient suspected of having acute cerebral vascular disease or a patient diagnosed with acute cerebral vascular disease,
wherein the sample derived from the patient is collected from the patient within 48 hours after the onset of symptoms of acute cerebral vascular disease in the providing step.

11. The method according to any one of claims 1 to 10, wherein the step of determining the concentration of the soluble CLEC2 is performed by highly sensitive immunoassay, such as chemiluminescence immunoassay, electrochemiluminescence immunoassay, or fluorescence immunoassay.
